# EUROPÄISCHE PATENTSCHRIFT

(11) **EP 0 373 576 B1**
(45) Veröffentlichungstag und Bekanntmachung des Hinweises auf die Patenterteilung: **22.03.1995**
(21) Anmeldenummer: 89122881.9
(22) Anmeldetag: 12.12.1989
(51) Int. Cl.: C07K 7/06, C12Q 1/37

(54) **Rasch spaltbares Substrat für die HIV-Protease**
Easily cleavable substrate for HIV protease
Substrat légèrement clivable pour la protéase de VIH

(30) Priorität: 15.12.1988 DE 3842197
(43) Veröffentlichungstag der Anmeldung: 20.06.1990
(73) Patentinhaber: HOECHST AKTIENGESELLSCHAFT, 65926 Frankfurt am Main (DE)
(72) Erfinder: Mölling, Karin, Prof. Dr., D-1000 Berlin 33 (DE); Henke, Stephan, Dr., D-6238 Hofheim am Taunus (DE); Breipohl, Gerhard, Dr., D-6000 Frankfurt am Main (DE); König, Wolfgang, Dr., D-6238 Hofheim am Taunus (DE)

(56) Entgegenhaltungen:
- EP-A- 0 271 865
- HELVETICA CHIMICA ACTA. vol. 66, no. 4, 1983, BASEL CH Seiten 1241 - 1252;Vasella,A. and Voeffray,R: "Synthesis of D- and L-5-Oxaproline and of a newCaptropril Analogue"
- CELL. vol. 54, 29 Juli 1988, CAMBRIDGE, NA US Seiten 3053 - 3058; Schneider,J.and Kent,S.B: "Enzymatic activity of a synthetic 99 residue proteincorresponding to theputative HIV-1 Protease"

## Beschreibung

Die Erfindung betrifft Hepta-Peptide bis Nona-Peptide sowie deren Verwendung zum Auffinden und Nachweis der HIV-Protease und zum Test möglicher Hemmer der HIV-Protease.

Die HIV-Protease, eine Retrovirus-Protease spaltet gag- und gag-pol-Precursor-Proteine von AIDS-Viren in die funktionellen Proteine. Diese korrekte Spaltung ist für die Infektion mit AIDS notwendig [N.E. Kohl, E.A. Emini, W.A. Schleif, L.J. Davis, J.C. Heimbach, R.A.F. Dixon, E.M. Scolnick und I.S. Sigal, Proc. Natl. Acad. Sci. USA 85 (1988) 4686-4690]. Die Protease ist in der 5′-Region des pol-Gens kodiert (z.B. HTLV-III-pol-(69-167)). Sie ist eine spezifische Aspartyl-Protease, die sich mit dem Aspartyl-Protease-Hemmer Pepstatin hemmen läßt. Gute zellgängige Inhibitoren dieser HIV-Protease haben Bedeutung für die Behandlung von AIDS-infizierten Patienten.

Das Enzym besteht lediglich aus 99 Aminosäuren und spaltet sich offensichtlich selbst durch Hydrolyse der beiden Phe-Pro-Bindungen in Position 68-69 bzw. 167-168 aus dem pol-Gen heraus [M.C. Graves, J.J. Lim, E.P. Heimer und R.A. Kramer, Proc. Natl. Acad. Sci. USA 85 (1988) 2449-2453; J. Hansen, S. Billich, T. Schulze, S. Sukrow und K. Moelling, The EMBO J. 7 (1988) 1785-1791; E.P. Lillehoj, F.H.R. Salazar, R.J. Mervis, M.G. Raum, H.W. Chan, N. Ahmad und S. Venkatesan, J. Virology 62 (1988) 3053-3058; J. Schneider und S.B.H. Kent, Cell 54 (1988) 363-368].

Als Substrate für die HIV-Protease dienten bislang folgende Peptide, die an den bezeichneten Stellen gespalten wurden [J. Schneider und S.B.H. Kent, Cell 54 (1988) 363-368]: 
RRSNQVSQNYPIVQNIQGRR

GHKARVLAEAMSQVTNSATIMMQRGNFRNQRK

DRQGTVSFNFPQVTLWQRPL

RRQIGATLNFPISPIETVRRa
[P.L. Darke, R.F. Nutt, S.F. Brady, V.M. Garsky, T.M. Ciccarone, C.-T. Leu, P. K. Lumma, R.M. Freidinger, D.F. Veber und I.S. Sigal, Biochem. Biophys. Res. Commun. 156 (1988) 297-303]: 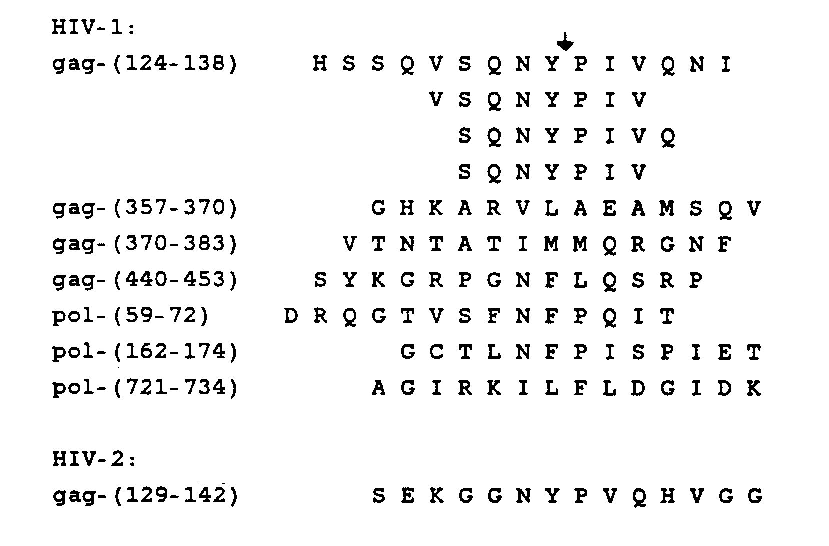
HIV-1:
gag-(124-138) H S S Q V S Q N Y P I V Q N I
V S Q N Y P I V
S Q N Y P I V Q
S Q N Y P I V
gag-(357-370) G H K A R V L A E A M S Q V
gag-(370-383) V T N T A T I M M Q R G N F
gag-(440-453) S Y K G R P G N F L Q S R P
pol-(59-72) D R Q G T V S F N F P Q I T
pol-(162-174) G C T L N F P I S P I E T
pol-(721-734) A G I R K I L F L D G I D K

HIV-2:
gag-(129-142) S E K G G N Y P V Q H V G G
Eine ähnliche Protease aus dem avian sarcoma-Leukosis virus spaltet ebenfalls die Tyr-Pro-Bindung [M. Kotler, R.A. Katz, W. Danho, J.Leis und A.M. Skalka, Proc. Natl. Acad. Sci. USA 85 (1988) 4185-4189]: 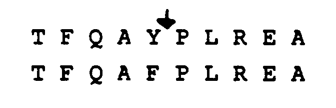
Es wurde auch bereits untersucht, wie sich die Eigenschaften von Peptiden - unter anderem auch HIV-1 Protease - ändern, wenn in ihnen natürliche Bausteine durch unnatürliche ersetzt werden (vgl. Helvetica Chimica Acta 66/4 (1988), 1241-1252, Cell 54 (1988), 363-368).

Überraschenderweise wurden nun gefunden, daß Substitution von Pro durch 5-Oxaprolin in diesen Sequenzen zu Substraten führt, die wesentlich schneller von der HIV-Protease gespalten werden. Man kommt mit diesen Substraten also schneller zu einem Ergebnis und benötigt weniger der schwer zugänglichen und teuren HIV-Proteasen.

Die Erfindung betrifft Hepta-Peptide bis Nona-Peptide der allgemeinen Formel I

A - B - C - D - E - Opr - F - G - H (I)

in welcher
- A: fehlen kann oder für Valin, Isoleucin oder Threonin steht,
- B: Serin, Threonin oder Phenylalanin,
- C: Leucin, Glutamin oder Phenylalanin
- D: Asparagin oder Alanin,
- E: Tyrosin oder Phenylalanin,
- Opr: den Rest von Isoxazolidin-3-carbonsäure (= 5-Oxaprolin),
- F: Isoleucin, Leucin, Valin, Threonin oder Glutamin,
- G: Valin, Isoleucin, Serin oder Arginin und
- H: fehlen kann oder Glutamin, Glutaminsäure, Prolin oder Threonin bedeuten,
sowie deren Salze.

Bevorzugt sind Peptide der Formel I, in welcher
- A: fehlt oder für Valin steht,
- B: Serin,
- C: Glutamin oder Phenylalanin,
- D: Asparagin,
- E: Tyrosin oder Phenylalanin,
- F: Isoleucin oder Glutamin,
- G: Valin oder Isoleucin und
- H: fehlt oder Glutamin bedeuten.

Insbesondere seien Peptide der Formel I genannt, in welcher
- A: fehlt,
- B: Serin,
- C: Phenylalanin,
- D: Asparagin,
- E: Phenylalanin,
- F: Glutamin,
- G: Isoleucin bedeuten und
- H: fehlt.

Als Salze kommen insbesondere Alkali- und Erdalkalisalze, Salze mit Aminen und Salze mit anorganischen oder organischen Säuren, wie beispielsweise Salzsäure, Bromwasserstoffsäure, Schwefelsäure, Maleinsäure, Essigsäure, Trifluoressigsäure oder Fumarsäuren in Betracht.

Die Erfindung betrifft weiterhin ein Verfahren zur Herstellung von Peptiden der Formel I, das dadurch gekennzeichnet ist, daß man
a) ein Fragment mit C-terminaler freier Carboxylgruppe oder dessen aktiviertes Derivat mit einem entsprechenden Fragment mit N-terminaler freier Aminogruppe umsetzt oder
b) das Peptid stufenweise aufbaut, in der nach (a) oder (b) erhaltenen Verbindung gegebenenfalls eine oder mehrere zum Schutz anderer Funktionen temporär eingeführte Schutzgruppen abspaltet, und die so erhaltenen Peptide der Formel I gegebenenfalls in ihr Salz überführt.

Die Peptide der vorliegenden Erfindung wurden nach allgemein bekannten Methoden der Peptidchemie, siehe z.B. Houben-Weyl, Methoden der organischen Chemie, Band 15/2, bevorzugt mittels Festphasensynthese wie z.B. von B. Merrifield, J.Am.Chem.Soc. 85, 2149 (1963), R. C. Sheppard Int.J.Peptide Protein Res. 21, 118 (1983) oder in EP-A-231 752 (HOE 86/F 099J) beschrieben oder in der europäischen Patentanmeldung Nr. 88 111011.8 (HOE 87/F 204) vorgeschlagen oder durch äquivalente bekannte Methoden hergestellt. Als α-Aminoschutzgruppe werden Urethanschutzgruppen wie z.B. die tert.-Butyloxycarbonyl(Boc)- oder Fluorenylmethyloxycarbonyl(Fmoc)-Schutzgruppe verwendet. Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. T.W.Greene, "Protective Groups in Organic Synthesis" oder EP-A-231 752) zusätzlich geschützt.

Die erfindungsgemäßen Peptide finden Verwendung beim Auffinden und Nachweis der HIV-Protease und der Austestung von möglichen Hemmern der HIV-Protease. Sie zeichnen sich dadurch aus, daß sie sehr schnell von der HIV-Protease gespalten werden wie nachfolgende Spaltungsversuche zeigen, für die verschiedenen Chargen einer klonierten HIV-Protease-Lösung verwendet wurden.

### A) Allgemeine Vorschrift für die Austestung von Inhibitoren der HIV-Protease mit Ser-Phe-Asn-Phe-Opr-Gln-Ile

a) Herstellung der Substratlösung:
   2 mg Ser-Phe-Asn-Phe-Opr-Gln-Ile werden in 1 ml MGTE15-Puffer gelöst (evtl. Anwendung von Ultraschall) und anschließend über einen Sterilfilter (0,45 »m) filtiert.
b) Herstellung der Inhibitorlösung:
   Vom Inhibitor werden das 2.5-fache der gewünschten Molarität je ml Lösung eingewogen und mit DMSO (10% des Endvolumens) gelöst. Man verdünnt mit MGTE15-Puffer bis zum Endvolumen und filtriert über Sterilfilter (0,45 »m).
c) Herstellung der Proteaselösung:
   5 »l der HIV-Proteaselösung werden nach Bedarf mit MGTE25-Puffer verdünnt.
d) Testdurchführung:
   Man pipettiert je 10 »l der Substratlösung in Reagenzgläser (16x100) mit Schraubdeckel. Zum Blindversuch werden 10 »l MGTE15-Puffer, der 10 % DMSO enthält, pipettiert. Die übrigen Reagenzgläser werden mit je 10 »l der Inhibitorlösungen versetzt. Man inkubiert 5-10 Minuten bei 37°C und gibt dann zu jeder Probe 5 »l der Proteaselösung. Nach 2 Stunden Reaktion bei 37°C werden von jeder Probe 10 oder 20 »l (je nach Empfindlichkeit des HPLC-Gerätes) abpipettiert, in Mikrovials gefüllt und mit 120 »l des HPLC-Laufmittels verdünnt.
e) Bedingungen für die HPLC-Analyse:
   Laufmittelsystem: 80% 0,1 M Phosphorsäure pH 2,5
   20% (w/w) Acetonitril
   Säule: Merck ®LICHROSORB RP18 (5 »m) 250x4
   Fluß: 1ml/min
   Temperatur der Säule: 42°C
   Detektorparameter: 215 nm, 0,08 AUF, 18,2°C
   Analysenzeit: 11 Minuten
   Retentionszeit des Substrates: 8,1 Minuten
   Retentionszeit des N-terminalen Tetrapeptids: 3,9 Minuten
f) Benötigte Lösungsmittel:
   1) MGTE15-Puffer:
      20 mM Morpholinoethansulfonsäure (MES)
      15 % (w/v) Glycerin
      0,1% (v/v) Triton X 100
      5 mM EDTA
      0,5 M NaCl
      1 mM Phenylmethylsulfonylfluorid (PMSF)
   2) MGTE25-Puffer:
      Zusammensetzung ähnlich wie beim MGTE15-Puffer mit folgender Abweichung:
      25 % (w/v) Glycerin,
      zusätzlich 1 mM Dithiothreit (DTT)
   In einen Erlenmeyerkolben wiegt man MES, EDTA, NaCl, DTT und PMSF ein, löst in wenig Wasser und stellt auf pH 6 ein. In einen Meßkolben wiegt man die entsprechende Menge Glycerin ein und pipettiert Triton X 100 dazu. Man überführt die wäßrige Lösung in den Meßkolben und füllt mit Wasser auf.
   3) HPLC-Laufmittel:
      Man stelle sich aus ortho-Phosphorsäure (FLUKA puriss. p.a.) eine 0,1 M Lösung her. Mit Triethylamin (FLUKA puriss. p.a.) wird diese Lösung genau auf pH 2,5 eingestellt. Das Gewicht der Lösung wird bestimmt und die entsprechende Menge Acetonitril (Abzug!) zugewogen. Gut durchmischen und ca.5 Minuten mit Helium 5,0 entgasen.
g) Auswertung:
   Unter den hier gewählten Bedingungen trennen sich die Heptapeptide von dem bei der enzymatischen Spaltung entstehenden N-terminalen Tetrapeptid. Der %-Gehalt des Tetrapeptid-peaks in Bezug auf Summe Tetrapeptid + Heptapeptid entspricht der Spaltrate.

### B) Durchführung der HIV-Protease-Kinetik (Tab. 1-4)

10 »l Substratlösung (2-4 mg/ml) in MGTE15-Puffer und 10 »l MGTE15-Puffer, der 10 % Dimethylsulfoxid enhält,werden vereinigt und 5-10 Minuten bei 37°C inkubiert. Dazu gibt man 5 »l der HIV-Protease-Lösung in MGTE25-Puffer und schüttelt bei 37°C. Nach 5, 10, 20, 40, 80, 160 und 360 Minuten werden je 2 »l entnommen und in 50 »l HPLC-Laufmittel pipettiert (stoppt die Reaktion). Mit der Mikroliterspritze wird die gesamte Menge auf die HPLC-Säule aufgegeben. HPLC-Bedingungen wie in A).

### C) Durchführung der HIV-Protease-Kinetik (Tab. 6)

10 »l Substratlösung (2 mg/ml) in MGTE 15-puffer und 10 »l MGTE15-Puffer werden vereinigt und 5-10 Minuten bei 37 °C inkubiert. Dazu gibt man 5 »l der HIV-Protease Nr. II (1:4 mit MGTE25-Puffer verdünnt) und schüttelt bei 37°C. Weitere Durchführung wie in B).

### HPLC-Bedingungen:

Säule: Merck HIBAR ®LICHROSORP RP8 (7 »m) 250x4
Fluß: 1 ml/min für SFNFPQI und SFNFOQI
0,5 ml/min für VSQNYPIVQ und VSQNYOIVQ.
Sonst wie in Ae).

| | | |
|---|---|---|
| Retentionszeiten für | SFNFPQI: | 6,7 min |
| | SFNFOQI: | 6,5 min |
| | SFNF: | 3,7 min |
| | VSQNYPIVQ: | 5,3 min |
| | VSQNYOIVQ: | 5,5 min |
| | VSQNY: | 4,6 min. |

Tabelle 1 und 2 zeigen die Spaltung von Ser-Phe-Asn-Phe-Pro-Gln-Ile (SFNFPQI) bzw. Ser-Phe-Asn-Phe-Opr-Gln-Ile (SFNFOQI) unter gleichen Bedingungen. Die Spaltung der Peptide in das N-terminale Tetrapeptid und das C-terminale Tripeptid wird mit HPLC verfolgt. Während das Pro- enthaltende Hepta-Peptid nach 5 Minuten Spaltzeit zu etwa 4 % gespalten war (Tab. 1), war das Opr-enthaltende Heptapeptid in der gleichen Zeit bereits zu 40-43 % gespalten (Tab. 2). Die Spaltung geht also etwa 10 mal so schnell mit den erfindungsgemäßen Substraten. Tabelle 3 und 4 zeigen, daß schon stark verdünnte HIV-Protease-Lösungen mit den neuen Substraten zu guten Spalt-Raten führen. Daß die Spaltung dieser Opr-haltigen Sequenzen auch gehemmt werden kann, zeigt Tabelle 5, aus der hervorgeht, daß Pepstatin A die HIV-Protease mit einem IC₅₀-Wert von etwa 10⁻⁶M hemmt. Tabelle 6 zeigt einen direkten Vergleich der bereits oben beschriebenen Heptapeptide mit den Nonapeptiden Val-Ser-Gln-Asn-Tyr-Pro-Ile-Val-Gln (VSQNYPIVQ) und Val-Ser-Gln-Asn-Tyr-Opr-Ile-Val-Gln (VSQNYOIVQ).

**Tabelle 1**

| Spaltung von Ser-Phe-Asn-Phe-Pro-Gln-Ile (4 mg/ml) mit HIV-Protease Nr. II (5 »l) | | |
|---|---|---|
| Zeit [min] | % Spaltung | |
| | Versuch A | Versuch B |
| 5 | 4,00 | 4,15 |
| 10 | 8,19 | 7,54 |
| 20 | 14,57 | 14,35 |
| 40 | 23,40 | 23,00 |
| 80 | 39,43 | 37,68 |
| 160 | 58,39 | 57,11 |
| 320 | 70,16 | 72,51 |

**Tabelle 2**

| Spaltung von Ser-Phe-Asn-Phe-Opr-Gln-Ile (4 mg/ml) mit HIV-Protease Nr. II (5 »l) | | |
|---|---|---|
| Zeit [min] | % Spaltung | |
| | Versuch A | Versuch B |
| 5 | 40,43 | 43,32 |
| 10 | 62,10 | 65,06 |
| 20 | 81,56 | 84,39 |
| 40 | 95,98 | 95,79 |
| 80 | 98,82 | 98,76 |
| 160 | 100,00 | 100,00 |
| 320 | 99,48 | 99,55 |

**Tabelle 3**

| Spaltung von Ser-Phe-Asn-Phe-Opr-Gln-Ile (2 mg/ml) mit verdünnter HIV-Protease Nr. II (5 »l) | | | | |
|---|---|---|---|---|
| Zeit [min] | % Spaltung | | | |
| | 1:10 | 1:20 | 1:30 | 1:40 |
| 5 | 9,71 | 4,92 | 3,42 | 2,57 |
| 10 | 17,29 | 9,16 | 6,12 | 4,50 |
| 20 | 29,13 | 15,96 | 10,50 | 7,63 |
| 40 | 48,34 | 27,23 | 17,82 | 12,57 |
| 80 | 70,56 | 44,82 | 30,94 | 21,82 |
| 160 | 88,07 | 64,12 | 48,87 | 35,59 |
| 320 | 90,74 | 70,52 | 57,99 | 43,78 |

**Tabelle 4**

| Umsetzung von Ser-Phe-Asn-Phe-Opr-Gln-Ile (2 mg/ml) nach 120 Minuten in Abhängigkeit verschiedener Konzentrationen der HIV-Protease Nr. II | | | | |
|---|---|---|---|---|
| | 1:10 (0,1) | 1:20 (0,05) | 1:30 (0,033) | 1:40 (0,025) |
| Umsetzung [%] | 82,5 | 57,0 | 42,0 | 30,0 |

**Tabelle 5**

| HIV-Protease-Test mit Ser-Phe-Asn-Phe-Opr-Gln-Ile (2 mg/ml) und HIV-Protease Nr. I (1:10) | | | |
|---|---|---|---|
| | Konz. (M) | Spaltung (%) | IC₅₀ |
| Blindwert | | 33,62 | |
| Pepstatin A | 10⁻⁴ | 1,25 | |
| Pepstatin A | 10⁻⁵ | 4,73 | |
| Pepstatin A | 10⁻⁶ | 18,22 | ca. 10⁻⁶ M |
| Pepstatin A | 10⁻⁷ | 25,09 | |

**Tabelle 6**

| Vergleich der Spaltgeschwindigkeit mehrerer Substrate (2 mg/ml) mit HIV-Protease Nr. II (1:4) | | | | |
|---|---|---|---|---|
| Zeit [min] | SFNFPQI | SFNFOQI | VSQNYPIVQ | VSQNYOIVQ |
| | (% Tetrapeptid = % Spaltung) | | | |
| 5 | 0,25 | 30,09 | 3,66 | 10,27 |
| 10 | 4,68 | 47,35 | 6,53 | 20,72 |
| 20 | 7,01 | 69,58 | 11,31 | 40,47 |
| 40 | 11,34 | 89,92 | 20,32 | 60,50 |
| 80 | 19,50 | 100,00 | 31,49 | 88,98 |
| 160 | 34,19 | 100,00 | 55,00 | 100,00 |
| 320 | 48,18 | 100,00 | 62,48 | 100,00 |

### Beispiel 1:

### Synthese von Ser-Phe-Asn-Phe-Opr-Gln-Ile

Das Zielpeptid wurde mit einem Peptid-Synthesizer Modell 430 A der Fa. Applied Biosystems unter Verwendung der Fmoc-Methode an einem mit Fmoc-Ile-OH veresterten p-Benzyloxybenzylalkohol-Harz der Fa. Novabiochem (Beladung ca. 0,5 mmol/g Harz) stufenweise aufgebaut. Es wurde 1 g des Harzes eingesetzt und die Synthese mit Hilfe eines für die Fmoc-Methode modifizierten Synthese-Programmes durchgeführt.

Man verwendet folgende Aminosäure-Derivate: Fmoc-Gln-OH, Fmoc-Opr-OH, Fmoc-Phe-OObt, Fmoc-Asn-OH und Fmoc-Ser(tBu)-OObt. Zur Synthese von Fmoc-Opr-OH wurde H-Opr-OtBu nach der Methode von Vasella et al. (J.C.S. Chem. Comm. 1981, 97-98) synthetisiert und mit Fmoc-OSu in Dioxan/Wasser (1:1) in Gegenwart von NaHCO₃ umgesetzt. Die anschließende Spaltung des tert.-Butylesters mit Trifluoressigsäure liefert Fmoc-Opr-OH.

In die Cartridges des Synthesizers wurden jeweils 1 mmol der Aminosäurederivate mit freier Carboxylgruppe zusammen mit 0,95 mmol HOObt eingewogen. Die Voraktivierung dieser Aminosäuren erfolgte direkt in den Cartridges durch Lösen in 4 ml DMF und Zugabe von 2 ml einer 0,55 molaren Lösung von Diisopropylcarbodiimid in DMF. Die HOObt-Ester der anderen Aminosäuren wurden in 6 ml NMP gelöst und dann ebenso wie die in situ voraktivierten Aminosäuren an das vorher mit 20 % Piperidin in DMF entblockierte Harz gekuppelt. Nach beendeter Synthese wurde das Peptid unter gleichzeitiger Entfernung der Seitenkettenschutzgruppen mit Trifluoressigsäure unter Verwendung von Thioanisol und Ethandithiol als Kationenfänger vom Harz abgespalten. Der nach Abziehen der Trifluoressigsäure erhaltene Rückstand wurde mehrfach mit Essigester digeriert und zentrifugiert.

Der verbliebene Rückstand wurde an einem alkylierten Dextrangel mit 10 %iger Essigsäure chromatographiert. Die das reine Peptid enthaltende Fraktion wurde vereinigt und gefriergetrocknet.
Massenspektrum (FAB): 854 (M+H⁺)
Aminosäureanalyse Asp: 0,98; Ser: 0,80; Glu: 1,00; Ile: 1,05; Phe: 2,10; NH₃: 1,76.

### Beispiel 2:

### Synthese von H-Ser-Phe-Asn-Phe-Pro-Gln-Ile-OH

Die Synthese erfolgte mit einem halbautomatischen Peptidsynthesizer Modell SP 640 der Fa. Labortec unter Benutzung von N_{α}-Fmoc geschützten Aminosäuren. Es wurden 5 g Fmoc-Ile-benzyloxybenzylalkohol-Harz (Beladung 0,43 mMol/g) eingesetzt. Dabei wurden folgende Syntheseschritte zyklisch durchgeführt:
1. Abspaltung der Fmoc-Schutzgruppe mit 20% Piperidin/DMF (2mal 50 ml),
2. Waschen des Harzes mit DMF (7mal 60 ml),
3. Aufkupplung der als HOBt-Ester in situ voraktivierten Fmoc-Aminosäure (Voraktivierung mit Diisopropylcarbodiimid in DMF) (5 Äquivalente Aminosäure, HOBt und Carbodiimid; 0,15 Äquivalente HOObt als Indikator für Fortschreiten der Reaktion),
4. Waschen mit DMF (5mal 60 ml)
Nach Aufkupplung von Fmoc-Pro-OH (5) wurden 10% des Harzes und nach Aufkupplung von Fmoc-Phe-OH (2) weitere 20% des Harzes für separate Abspaltungen entnommen.
Nach beendeter Synthese des Heptapeptides erhielt man nach Fmoc-Abspaltung, Waschen des Harzes mit DMF, DCM und MTB-Ether 4,2 g H-Ser(tBu)-Phe-Asn-Phe-Pro-Gln-Ile-Harz.

Das Peptid wurde durch Behandlung mit 95% Trifluoressigsäure/H₂O vom Harz gespalten (2,5 h bei Raumtemperatur). Das Harz wurde abfiltriert und mit 95% TFA gewaschen. Das Filtrat wurde zur Trockene eingeengt, mit Ether verrührt und im Hochvakuum getrocknet. Ausbeute an Rohpeptid 1,13 g. Die Reinigung erfolgte an einem alkylierten Dextrangel mit 10% wäßriger Essigsäure als Elutionsmittel. Die das reine Peptid enthalten Fraktionen wurden vereinigt und gefriergetrocknet.
Ausbeute: 323 mg
MS(FAB): 852 (M+H⁺)
Aminosäureanalyse: Asp:1,01; Ser:0,75; Glu:0,97; Pro:1,03; Ile:1,00; Phe:2.00.

### Beispiel 3:

### Synthese von H-Val-Ser-Gln-Asn-Tyr-Pro-Ile-Val-Gln-OH

Die Synthese erfolgte an einem automatischen Peptidsynthesizer Modell 430A der Fa. Applied Biosystems unter Verwendung von isolierten Fmoc-Aminosäure-OObt Estern oder in situ aktivierten HOBt-Estern. Dabei wurden folgende Zyklen durchlaufen:
1. Abspalten von Fmoc mit 20% Piperidin in DMF
2. Waschen des Harzes mit NMP
3. Aufkuppeln der Fmoc-Aminosäure in NMP (Fmoc-Val-OObt, Fmoc-Ile-OObt, Fmoc-Pro-OObt, Fmoc-Tyr(tBu)-OObt, Fmoc-Ser(tBu)-OObt oder in DMF (als HOBt-Ester in situ mit Diisopropylcarbodiimid voraktivierte Aminosäuren Fmoc-Asn-OH, Fmoc-Gln-OH)
4. Waschen mit NMP
Die Synthese erfolgte an einem in der deutschen Patentanmeldung P 37 43 620.1 (HOE 87/F 386) beschriebenen Amidankerharz an das Fmoc-Glu-OtBu über die π-Carboxylgruppe gekuppelt war. Diese Harz liefert bei der Abspaltung mit Säure das C-terminale Glutamin. Es wurden 500 mg des Fmoc-Glu(NH-Anker)-OtBu Harzes eingesetzt.

Nach beendeter Synthese und Abspaltung von Fmoc erhielt man 817 mg Peptidharz. Das Peptid wurde durch Behandlung mit 95 % Trifluoressigsäure/H₂O vom Harz gespalten (3 h bei Raumtemperatur). Das Harz wurde abfiltriert und mit 95 % TFA gewaschen. Das Filtrat wurde zur Trockene eingeengt, mit Ether verrührt und im Hochvakuum getrocknet. Ausbeute an Rohpeptid 236 mg. Die Reinigung erfolgte an alkylierten Dextrangel mit 10% wäßriger Essigsäure als Elutionsmittel. Die das reine Peptid enthaltenden Fraktionen wurden vereinigt und gefriergetrocknet.
Ausbeute: 142 mg
MS(FAB): 1047 (M+H⁺)
Aminosäureanalyse: Asp:1,00; Ser:0,87; Glu:2,04; Pro:1,00; Val:1,96; Ile:0,86; Tyr:0,91.

### Beispiel 4:

### Synthese von H-Val-Ser-Gln-Asn-Tyr-Opr-Ile-Val-Gln-OH

Die Synthese erfolgte an einem automatischen Peptidsynthesizer Modell 430A der Fa. Applied Biosystems unter Verwendung von isolierten Fmoc-Aminosäure-OObt Estern oder in situ aktivierten HOBt Estern.
Dabei wurden folgende Zyklen durchlaufen:
1. Abspalten von Fmoc mit 20% Piperidin in DMF
2. Waschen des Harzes mit NMP
3. Aufkuppeln der Fmoc-Aminosäure in NMP (Fmoc-Val-OObt, Fmoc-Ile-OObt, Fmoc-Tyr(tBu)-OObt, Fmoc-Ser(tBu)-OObt oder in DMF (als HOBt-Ester in situ mit Diisopropylcarbodiimid voraktivierte Aminosäuren Fmoc-Opr-OH, Fmoc-Asn-OH, Fmoc-Gln-OH)
4. Waschen mit NMP
Die Synthese erfolgte an einem in der deutschen Patentanmeldung P 37 43 620.1 (HOE 87/F 386) beschriebenen Amidankerharz an das Fmoc-Glu-OtBu über die π-Carboxylgruppe gekuppelt war. Dieses Harz liefert bei der Abspaltung mit Säure das C-terminale Glutamin. Es wurden 500 mg des Fmoc-Glu(NH-Anker)-OtBu Harzes eingesetzt. Nach beendeter Synthese und Abspaltung von Fmoc erhielt man 820 mg Peptidharz. Das Peptid wurde durch Behandlung mit 95 % Trifluoressigsäure/H₂O vom Harz gespalten (3 h bei Raumtemperatur). Das Harz wurde abfiltriert und mit 95% TFA gewaschen. Das Filtrat wurde zur Trockene eingeengt, mit Ether verrührt und im Hochvakuum getrocknet. Ausbeute an Rohpeptid 260 mg. Die Reinigung erfolgte an einem alkylierten Dextrangel mit 10 % wäßriger Essigsäure als Elutionsmittel. Die das reine Peptid enthaltenden Fraktionen wurden vereinigt und gefriergetrocknet.
Ausbeute: 69 mg
MS(FAB): 1047 (M+H⁺)
Aminosäureanalyse: Opr:1,01 Asp:1,00; Ser:0,88; Glu:2,07; Val:1,88; Ile:0,86; Tyr:0,44.

## Patentansprüche (Patentansprüche für folgende(n) Vertragsstaat(en): AT, BE, CH, DE, FR, GB, IT, LI, LU, NL, SE)

1. Hepta-Peptid bis Nona-Peptid der Formel I
A - B - C - D - E - Opr - F - G - H (I)
in welcher
A fehlen kann oder für Valin, Isoleucin oder Threonin steht,
B Serin, Threonin oder Phenylalanin,
C Leucin, Glutamin oder Phenylalanin
D Asparagin oder Alanin,
E Tyrosin oder Phenylalanin,
Opr den Rest von Isoxazolidin-3-carbonsäure (= 5-Oxaprolin),
F Isoleucin, Leucin, Valin, Threonin oder Glutamin,
G Valin, Isoleucin, Serin oder Arginin und
H fehlen kann oder Glutamin, Glutaninsäure, Prolin oder Threonin bedeuten,
sowie dessen Salze.

2. Peptid der Formel I, gemäß Anspruch 1, in welcher
A fehlt oder für Valin steht,
B Serin,
C Glutamin oder Phenylalanin,
D Asparagin,
E Tyrosin oder Phenylalanin,
F Isoleucin oder Glutamin,
G Valin oder Isoleucin und
H fehlt oder Glutamin bedeuten,
sowie dessen Salze

3. Peptid der Formel I gemäß einem oder mehreren der Ansprüche 1 bis 2, in welcher
A fehlt,
B Serin,
C Phenylalanin,
D Asparagin,
E Phenylalanin,
F Glutamin,
G Isoleucin bedeuten und
H fehlt,
sowie dessen Salze.

4. Verfahren zur Herstellung eines Peptids der Formel I, gemäß einem oder mehreren der Ansprüche 1 bis 3, dadurch gekennzeichnet, daß man
a) ein Fragment mit C-terminaler freier Carboxylgruppe oder dessen aktiviertes Derivat mit einem entsprechenden Fragment mit N-terminaler freier Aminogruppe umsetzt oder
b) das Peptid stufenweise aufbaut, in der nach (a) oder (b) erhaltenen Verbindung gegebenenfalls eine oder mehrere zum Schutz anderer Funktionen temporär eingeführte Schutzgruppen abspaltet, und die so erhaltenen Peptide der Formel I gegebenenfalls in ihr Salz überführt.

5. Verwendung eines Peptides der Formel I, gemäß einem oder mehreren der Ansprüche 1 bis 3, zum Auffinden und Nachweis der HIV-Protease.

6. Verwendung eines Peptides der Formel I, gemäß einem oder mehreren der Ansprüche 1 bis 3, zur Austestung von möglichen Hemmern der HIV-Protease.

## Patentansprüche (Patentansprüche für folgende(n) Vertragsstaat(en): ES, GR)

1. Verfahren zur Herstellung eines Hepta-Peptids bis Nona-Peptids der Formel I
A - B - C - D - E - Opr - F - G - H (I)
in welcher
A fehlen kann oder für Valin, Isoleucin oder Threonin steht,
B Serin, Threonin oder Phenylalanin,
C Leucin, Glutamin oder Phenylalanin
D Asparagin oder Alanin,
E Tyrosin oder Phenylalanin,
Opr den Rest von Isoxazolidin-3-carbonsäure (= 5-Oxaprolin),
F Isoleucin, Leucin, Valin, Threonin oder Glutamin,
G Valin, Isoleucin, Serin oder Arginin und
H fehlen kann oder Glutamin, Glutaminsäure, Prolin oder Threonin bedeuten,
sowie dessen Salze, dadurch gekennzeichnet, daß man
a) ein Fragment mit C-terminaler freier Carboxylgruppe oder dessen aktiviertes Derivat mit einem entsprechenden Fragment mit N-terminaler freier Aminogruppe umsetzt oder
b) das Peptid stufenweise aufbaut, in der nach (a) oder (b) erhaltenen Verbindung gegebenenfalls eine oder mehrere zum Schutz anderer Funktionen temporär eingeführte Schutzgruppen abspaltet, und die so erhaltenen Peptide der Formel I gegebenenfalls in ihr Salz überführt.

2. Verfahren gemäß Anspruch 1, dadurch gekennzeichnet, daß man ein Peptid der Formel I herstellt, in welchem
A fehlt oder für Valin steht,
B Serin,
C Glutamin oder Phenylalanin,
D Asparagin,
E Tyrosin oder Phenylalanin,
F Isoleucin oder Glutamin,
G Valin oder Isoleucin und
H fehlt oder Glutamin bedeuten,

3. Verfahren gemäß Anspruch 1 und/oder 2, dadurch gekennzeichnet, daß man ein Peptid der Formel I herstellt, in welchem
A fehlt,
B Serin,
C Phenylalanin,
D Asparagin,
E Phenylalanin,
F Glutamin,
G Isoleucin bedeuten und
H fehlt,

4. Verwendung eines nach einem der Ansprüche 1 bis 3 hergestellten Peptids der Formel I zum Auffinden und Nachweis der HIV-Protease.

5. Verwendung eines nach einem der Ansprüche 1 bis 3 hergestellten Peptids der Formel I zur Austestung von möglichen Hemmern der HIV-Protease.

## Claims (Claims for the following Contracting State(s): AT, BE, CH, DE, FR, GB, IT, LI, LU, NL, SE)

1. A hepta-peptide to nona-peptide of the formula I
A - B - C - D - E - Opr - F - G - H (I)
in which
A can be absent or represents valine, isoleucine or threonine,
B denotes serine, threonine or phenylalanine,
C denotes leucine, glutamine or phenylalanine,
D denotes asparagine or alanine,
E denotes tyrosine or phenylalanine,
Opr denotes the radical of isoxazolidine-3-carboxylic acid (= 5-oxaproline),
F denotes isoleucine, leucine, valine, threonine or glutamine,
G denotes valine, isoleucine, serine or arginine and
H can be absent or denotes glutamine, glutamic acid, proline or threonine,
as well as the salts thereof.

2. A peptide of the formula I as claimed in claim 1, in which
A is absent or represents valine,
B denotes serine,
C denotes glutamine or phenylalanine,
D denotes asparagine,
E denotes tyrosine or phenylalanine,
F denotes isoleucine or glutamine,
G denotes valine or isoleucine and
H is absent or denotes glutamine.
as well as the salts thereof,

3. A peptide of the formula I as claimed in one or more of claims 1 to 2, in which
A is absent,
B denotes serine,
C denotes phenylalanine,
D denotes asparagine,
E denotes phenylalanine,
F denotes glutamine,
G denotes isoleucine and
H is absent,
as well as the salts thereof.

4. A process for the preparation of a peptide of the formula I as claimed in one or more of claims 1 to 3, which comprises
a) reacting a fragment with a C-terminal free carboxyl group or the activated derivative thereof with a corresponding fragment with an N-terminal free amino group or
b) synthesizing the peptide stepwise, eliminating one or more protective groups temporarily introduced to protect other functionalities where appropriate in the compound obtained as in (a) or (b), and converting the peptides of the formula I obtained in this way into the salt thereof where appropriate.

5. The use of a peptide of the formula I as claimed in one or more of claims 1 to 3, for locating and detecting HIV protease.

6. The use of a peptide of the formula I as claimed in one or more of claims 1 to 3, for testing possible inhibitors of HIV protease.

## Claims (Claims for the following Contracting State(s): ES, GR)

1. A process for the preparation of a hepta-peptide to nona-peptide of the formula I
A - B - C - D - E - Opr - F - G - H (I)
in which
A can be absent or represents valine, isoleucine or threonine,
B denotes serine, threonine or phenylalanine,
C denotes leucine, glutamine or phenylalanine,
D denotes asparagine or alanine,
E denotes tyrosine or phenylalanine,
Opr denotes the radical of isoxazolidine-3-carboxylic acid (= 5-oxaproline),
F denotes isoleucine, leucine, valine, threonine or glutamine,
G denotes valine, isoleucine, serine or arginine and
H can be absent or denotes glutamine, glutamic acid, proline or threonine,
as well as the salts thereof, which comprises
a) reacting a fragment with a C-terminal free carboxyl group or the activated derivative thereof with a corresponding fragment with an N-terminal free amino group or
b) synthesizing the peptide stepwise, eliminating one or more protective groups temporarily introduced to protect other functionalities where appropriate in the compound obtained as in (a) or (b), and converting the peptides of the formula I obtained in this way into the salt thereof where appropriate.

2. The process as claimed in claim 1, which comprises preparing a peptide of the formula I in which
A is absent or represents valine,
B denotes serine,
C denotes glutamine or phenylalanine,
D denotes asparagine,
E denotes tyrosine or phenylalanine,
F denotes isoleucine or glutamine,
G denotes valine or isoleucine and
H is absent or denotes glutamine.

3. The process as claimed in claim 1 and/or 2, which comprises preparing a peptide of the formula I in which
A is absent,
B denotes serine,
C denotes phenylalanine,
D denotes asparagine,
E denotes phenylalanine,
F denotes glutamine,
G denotes isoleucine and
H is absent.

4. The use of a peptide of the formula I prepared as claimed in one of claims 1 to 3 for locating and detecting HIV protease.

5. The use of a peptide of the formula I prepared as claimed in one of claims 1 to 3 for testing possible inhibitors of HIV protease.

## Revendications (Revendications pour l'(les) Etat(s) contractant(s) suivant(s): AT, BE, CH, DE, FR, GB, IT, LI, LU, NL, SE)

1. Heptapeptide à nonapeptide de formule I
A - B - C - D - E - Opr - F - G - H (I)
dans laquelle
A peut être absent ou représente le reste valine, isoleucine ou thréonine,
B représente le reste sérine, thréonine ou phénylalanine,
C représente le reste leucine, glutamine ou phénylalanine,
D représente le reste asparagine ou alanine,
E représente le reste tyrosine ou phénylalanine,
Opr représente le reste de l'acide isoxazolidine-3-carboxylique (= 5-oxaproline),
F représente le reste isoleucine, leucine, valine, thréonine ou glutamine,
G représente le reste valine, isoleucine, sérine ou arginine, et
H peut être absent ou représente le reste glutamine, acide glutamique, proline ou thréonine,
et sels de celui-ci.

2. Peptide de formule I selon la revendication 1, dans lequel
A est absent ou représente le reste valine,
B représente le reste sérine,
C représente le reste glutamine ou phénylalanine,
D représente le reste asparagine,
E représente le reste tyrosine ou phénylalanine,
F représente le reste isoleucine ou glutamine,
G représente le reste valine ou isoleucine et
H est absent ou représente le reste glutamine,
et sels de celui-ci.

3. Peptide de formule I selon la revendication 1 ou 2, dans lequel
A est absent,
B représente le reste sérine,
C représente le reste phénylalanine,
D représente le reste asparagine,
E représente le reste phénylalanine,
F représente le reste glutamine,
G représente le reste isoleucine et
H est absent,
et sels de celui-ci.

4. Procédé pour la préparation d'un peptide de formule I selon une ou plusieurs des revendications 1 à 3, caractérisé en ce que
a) on fait réagir un fragment à groupe carboxy C-terminal libre, ou un dérivé activé de celui-ci, avec un fragment correspondant à groupe amino N-terminal libre, ou
b) on fait la synthèse du peptide par étapes, et dans le composé obtenu selon a) ou b), on élimine éventuellement un ou plusieurs groupes protecteurs temporairement introduits pour la protection d'autres fonctions, et éventuellement on convertit en un de leurs sels les peptides de formule I ainsi obtenus.

5. Utilisation d'un peptide de formule I selon une ou plusieurs des revendications 1 à 3, pour la recherche et la détection de la protéase du HIV.

6. Utilisation d'un peptide de formule I selon une ou plusieurs des revendications 1 à 3, pour l'essai d'éventuels inhibiteurs de la protéase du HIV.

## Revendications (Revendications pour l'(les) Etat(s) contractant(s) suivant(s): ES, GR)

1. Procédé pour la préparation d'un heptapeptide à nonapeptide de formule I
A - B - C - D - E - Opr - F - G - H (I)
dans laquelle
A peut être absent ou représente le reste valine, isoleucine ou thréonine,
B représente le reste sérine, thréonine ou phénylalanine,
C représente le reste leucine, glutamine ou phénylalanine,
D représente le reste asparagine ou alanine,
E représente le reste tyrosine ou phénylalanine,
Opr représente le reste de l'acide isoxazolidine-3-carboxylique (= 5-oxaproline),
F représente le reste isoleucine, leucine, valine, thréonine ou glutamine,
G représente le reste valine, isoleucine, sérine ou arginine, et
H peut être absent ou représente le reste glutamine, acide glutamique, proline ou thréonine,
et de ses sels,
caractérisé en ce que
a) on fait réagir un fragment à groupe carboxy C-terminal libre, ou un dérivé activé de celui-ci, avec un fragment correspondant à groupe amino N-terminal libre, ou
b) on fait la synthèse du peptide par étapes, et dans le composé obtenu selon a) ou b), on élimine éventuellement un ou plusieurs groupes protecteurs temporairement introduits pour la protection d'autres fonctions, et éventuellement on convertit en un de leurs sels les peptides de formule I ainsi obtenus.

2. Procédé selon la revendication 1, caractérisé en ce que l'on prépare un peptide de formule I dans lequel A est absent ou représente le reste valine,
B représente le reste sérine,
C représente le reste glutamine ou phénylalanine,
D représente le reste asparagine,
E représente le reste tyrosine ou phénylalanine,
F représente le reste isoleucine ou glutamine,
G représente le reste valine ou isoleucine et
H est absent ou représente le reste glutamine.

3. Procédé selon la revendication 1 et/ou 2, caractérisé en ce que l'on prépare un peptide de formule I dans lequel
A est absent,
B représente le reste sérine,
C représente le reste phénylalanine,
D représente le reste asparagine,
E représente le reste phénylalanine,
F représente le reste glutamine,
G représente le reste isoleucine et
H est absent.

4. Utilisation d'un peptide de formule I préparé selon l'une des revendications 1 à 3, pour la recherche et la détection de la protéase du HIV.

5. Utilisation d'un peptide de formule I préparé selon l'une des revendications 1 à 3, pour l'essai d'éventuels inhibiteurs de la protéase du HIV.
